(19) **Europäisches Patentamt**
**European Patent Office**
**Office européen des brevets**

(11) **EP 1 660 641 B1**

(12) **EUROPEAN PATENT SPECIFICATION**

(45) Date of publication and mention of the grant of the patent:
**04.04.2012 Bulletin 2012/14**

(51) Int Cl.:
**C12N 5/00** (2006.01)

(21) Application number: **04738155.3**

(22) Date of filing: **18.08.2004**

(86) International application number:
**PCT/CH2004/000520**

(87) International publication number:
**WO 2005/021737 (10.03.2005 Gazette 2005/10)**

(54) **FOLLICULAR FLUID FOR PROLONGED GROWTH AND SURVIVAL OF CELLS FOR CELL THERAPIES**

FOLLIKULAR FLÜSSIGKEIT FÜR VERLÄNGERTES WACHSTUM UND ÜBERLEBEN DER ZELLEN FÜR ZELLTHERAPIEN

FLUIDE FOLLICULAIRE POUR LA CROISSANCE ET LA SURVIE PROLONGEE DES CELLULES POUR DES THERAPIES CELLULAIRES

(84) Designated Contracting States:
**AT BE BG CH CY CZ DE DK EE ES FI FR GB GR HU IE IT LI LU MC NL PL PT RO SE SI SK TR**

(30) Priority: **01.09.2003 EP 03405628**

(43) Date of publication of application:
**31.05.2006 Bulletin 2006/22**

(73) Proprietor: **Medicyte GmbH**
**69120 Heidelberg (DE)**

(72) Inventor: **HORISBERGER, Michel, André**
**CH-4123 Allschwil (CH)**

(74) Representative: **Simandi, Claus**
**Patentanwalt**
**Höhenstrasse 26**
**53773 Hennef / Bonn (DE)**

(56) References cited:
**US-A1- 2002 045 259     US-B1- 6 323 025**

- BERNARD J: "EFFECT OF FOLLICULAR FLUID AND ESTRADIOL ON THE LUTEINIZATION OF RAT GRANULOSA CELLS IN-VITRO" JOURNAL OF REPRODUCTION AND FERTILITY, vol. 43, no. 3, 1975, pages 453-460, XP008028622 ISSN: 0022-4251
- ABU-MUSA A ET AL: "Effect of human follicular fluid on sperm survival" CLINICAL AND EXPERIMENTAL OBSTETRICS AND GYNECOLOGY, vol. 28, no. 1, 2001, pages 40-42, XP008036452 ISSN: 0390-6663
- ANDERSEN M M ET AL: "PROTEIN COMPOSITION IN THE FLUID OF INDIVIDUAL BOVINE FOLLICLES" JOURNAL OF REPRODUCTION AND FERTILITY, JOURNALS OF REPRODUCTION AND FERTILITY LTD, GB, vol. 48, no. 1, September 1976 (1976-09), pages 109-118, XP008028634 ISSN: 0022-4251

SEQUENCE LISTING

    <110> OvaGen AG

    <120> prolonged growth and survival of cells for cell therapies

    <130> zb/20646

    <150> EP03405628.3
    <151> 2003-09-01

    <160> 8

    <170> PatentIn version 3.1

    <210> 1
    <211> 14
    <212> PRT
    <213> Sus sp.

    <400> 1

```
    Thr Glu Val Ser Ser Asn His Val Leu Ile Tyr Leu Asp Lys
    1               5                   10
```

    <210> 2

<211> 23
<212> PRT
<213> Sus sp.

<400> 2

Gln Gln Asn Ala Gln Gly Gly Phe Ser Ser Thr Gln Asp Thr Val Val
1               5                   10                  15

Ala Leu His Ala Leu Ser Lys
            20

<210> 3
<211> 13
<212> PRT
<213> Sus sp.

<400> 3

Met Val Ser Gly Phe Ile Pro Leu Lys Pro Thr Val Lys
1               5                   10

<210> 4
<211> 11
<212> PRT
<213> Sus sp.

<400> 4

Ser Ser Gly Ser Leu Leu Asn Asn Ala Ile Lys
1               5                   10

<210> 5
<211> 10
<212> PRT
<213> Sus sp.

<400> 5

Gln Thr Val Ser Trp Ala Val Thr Pro Lys
1               5                   10

<210> 6
<211> 7
<212> PRT
<213> Sus sp.

<400> 6

Gly Glu Ala Phe Thr Leu Lys
1               5

<210> 7

<211> 8
<212> PRT
<213> Sus sp.

<400> 7

```
Tyr Gly Ala Ala Thr Phe Thr Arg
1               5
```

<210> 8
<211> 8
<212> PRT
<213> Sus sp.

<400> 8

```
Asp Leu Lys Pro Ala Ile Val Lys
1               5
```

**Description**

TECHNICAL FIELD

Tissue engineering is the development of biological substitutes to restore, maintain, or improve tissue function. Specifically, tissue engineering is a method by which new living tissues are created in the laboratory to replace diseased or traumatised tissue.

BACKGROUND OF THE INVENTION

One strategy to regenerate tissue is to isolate specific cells from tissue, expand the isolated cells *in vitro,* and implant the expanded cells into the diseased or traumatised tissue so that the implanted cells proliferate *in vivo* and eventually replace or repair the tissue defect. This technique has been applied to a variety of cell types and tissue defects. Isolated cells could be either differentiated cells from specific tissues or undifferentiated progenitor cells (stem cells). In both cases, establishment of appropriate culture conditions for cell expansion is extremely important in order to maintain or improve their potential to regenerate structural and functional tissue equivalents.

A particular area of focus for the development of tissue regeneration techniques is the correction of defects in cartilaginous tissue. Unlike other tissues, cartilage has little ability to regenerate itself after trauma, disease or as a result of old age. This is due to the avascular nature of normal articular cartilage. Although damage to the superficial chondral plate generally does not heal, the subchondral bone is vascularised, therefore damage to this location does heal to a limited degree. The new cartilage that grows in place of the damaged, articular cartilage is called fibrocartilage. Fibrocartilage lacks the durability and more desirable mechanical properties of the original hyaline cartilage. People who suffer joint damage are thereafter predisposed to arthritic degeneration.

Several different approaches have been taken to repair cartilage tissue, including chondral shaving, subchondral drilling, and tissue auto/allografts. Other experimental approaches for articular cartilage repair consist in harvesting chondrocytes from a cartilage biopsy and seeding the chondrocytes directly onto a three dimensional transplantation matrix material before implantation of the graft into the damaged area. This technique results in high quality cartilage once regeneration is complete; however, it would require a large quantity of starting material to be harvested from the patient, resulting in increased patient trauma.

In other approaches chondrocytes are isolated from a biopsy, expanded in monolayer cultures until a sufficient number of cells are obtained and implanted into the damaged area of tissue. Also in these cases, the implantation requires first that the cells are either embedded in a gel or associated with a biodegradable polymer scaffold. The three dimensional nature of those matrices imparts structural integrity to the implant and provides rigid support for growth of the chondrocyte cells into cartilaginous tissue. Although this system has the advantage of requiring fewer cells as starting material, the cartilage obtained by this method is often of poor quality if the cells are harvested or obtained from skeletally mature donors (adults).

An other major problem in tissue engineering is the proper technique to keep living cells in culture for growing and proliferation. Cellular senescence is a process that prevents normal diploid mammalian cells from growing (proliferating) indefinitely in culture.

Today the vast majority of degenerative diseases are treated by drugs or symptomatic therapies due to a lack of available patient-compatible cells or tissues generated, for example, by one of the above mentioned method, whereby said tissue could replace damaged tissue or repair the lesions induced by a given disorder.

Other current cell-based therapeutic approaches being involved are either allogeneic cells derived from human embryonic stem (ES) cells or xenogeneic cells derived from pigs. Examples of these approaches for Parkinson's disease are differentiated human neurones (Geron) and foetal pig neural cells (Diacrin/GenVec). Although these strategies hold scientific promise, they suffer from major limitations. First, human ES cells raise substantial ethical issues since human embryos have to be destroyed to generate ES cells. Second, the use of pig cells suffers from potentially unknown issues involving the transmission of porcine-borne pathogens to human. Third, both of these strategies require the use of immuno-suppression.

US 6,323,025 (Univ. of New Mexico), discloses a method for delaying senescence by culturing cells in a culture medium comprising a "vitaletheine modulator", i.e. beta-alanyl-taurine and derivatives thereof.

There is at present a great need for an efficient method to derive multi-potential stem-like cells from patient's own somatic cells.

More and more animals, particularly livestock species, are being cloned by nuclear transfer of a nucleus from a somatic cell into enucleated oocytes. The method suffers from a low efficiency.

SUMMARY OF THE INVENTION

The object of the present invention is a technology to change the nuclear function of one type of highly specialised somatic cell, e.g. granulosa cells, into that of another type, via a novel pluripotent intermediate. The invention does not utilise embryonic or foetal tissues to accomplish the change in function and can be designed for individual patients using their own cells.

It has now been found, surprisingly, that the culturing of somatic cells in the presence of a serum containing follicular fluid or components of a follicular fluid prevents senescence of the cells. Moreover the somatic cells with a limited lifespan cultured *in vitro* in the presence of said components or said follicular fluid acquire some properties of stem cells and can be passaged continuously, but only in the continuing presence of follicular fluid or components from the follicular fluid.

The invention relates to a method for preventing senescence and death of somatic cells comprising culturing the cells in a medium and further containing follicular fluid or components thereof.

In particular the invention relates to said methods comprising the steps of

- culturing the cells in a medium;
- introducing a follicular fluid or components thereof into the culture medium and
- allowing indefinite proliferation of cells in repeated subcultures.

Serum-containing culture medium is widely used in the art. Typically the serum is foetal calf serum. Depending on the type of somatic cells it is also possible to use a culture medium free of serum but being fully equivalent, thereby avoiding side effects of foetal calf serum not being totally understood. Such equivalent serum-free culture media are also well known in the art, and contain, among inorganic salts in proper concentrations, usually growth hormones, e.g. bovine growth hormone, or other growth-supporting biochemical factors, and hormones.

Follicular fluid is obtained from ovaries, and is readily available in large quantities. Particular follicular fluid considered is porcine, bovine, ovine and equine follicular fluid. Preferred is porcine follicular fluid.

Follicular fluid may be purified or partially purified using standard purification methods, such as centrifugation and filtration over an inert filter. Further purification may be obtained by filtration over charcoal which is known to retain low molecular components and impurities. Components may be obtained by fractionation according to molecular mass and/or polarity.

Charcoal filtered follicular fluid is preferably used to prolong the life span of normal human diploid fibroblasts, whereas untreated follicular fluid is preferably used to establish and maintain granulosa cell lines. Follicular fluid charcoal filtered can be mixed with untreated follicular fluid, preferably in approximately one to one ratio, and the mixture is superior in activity to untreated follicular fluid as shown for the establishment of primary granulosa cell lines and for the proliferation of primary granulosa cell lines. An approximately 1:1 mixture of unfiltered follicular fluid and charcoal filtered follicular fluid is particularly effective in the method of the invention, and is therefore preferred.

Further preferred as components of follicular fluid in the inventive method are molecular size fractions > 30 kDa and the combined molecular size fractions > 50 kDa and < 3 kDa. The preferred high molecular size fractions contain peptides which correspond to amino acid sequences of the human alpha-2-macroglobulin precursor and to homologs of other species, and these are likewise preferred in the method of the invention. The members of this family of proteins are in the form of a homotetramer, which consists of two pairs of disulfide-linked chains. High molecular mass components of follicular fluid with growth promoting activity and matrix properties are preferred. Likewise low molecular mass components of follicular fluid with cell survival activity are preferred.

Mammalian somatic cells, which can be rescued from senescence and death and which can be de-differentiated in a manner that results in successful proliferation of the cells and maintenance of their differentiation potential are, for example, granulosa cells, dermal fibroblast cells, neurons, keratinocytes or hepatocytes, in particular granulosa cells, dermal fibroblast cells and neurons. The invention, however, is not restricted to these particular somatic cells, but is applicable to any type of somatic cells. According to the present invention, progenitor type (or stem cell-like) cells will be generated which then can be used to generate new tissue. Any cell type that can be isolated and expanded is usable to regenerate new tissue. Non-limiting examples include also endothelial cells, muscle cells, chondrocytes and melanocytes.

Senescence of somatic cells is a particular problem when such cells are frozen, stored, and used later for culturing. Culturing efficiency is drastically reduced in many types of somatic cells on freezing and storing. The efficiency of the method of the invention can be readily demonstrated on such thawed cells.

The invention further relates to the use of a follicular fluid or components thereof for preventing senescence and death in a manner that results in successful proliferation of the cells and maintenance of their differentiation potential.

Based on said use the present invention provides a novel method for maintaining infinite proliferative potential of adult somatic cells. These cells can be used as a source of differentiating cells which have medical applications for treatment of degenerative diseases by "therapeutic cloning".

The present invention can also increase the efficiency of nuclear transfer by providing unlimiting amount of cells which are re-programmed toward pluripotency.

Immortalisation of somatic cells is usually obtained by transfection of a cultured cell with an expression vector for telomerase enzyme. The present invention circumvents the transfection method by providing the cell with a proper environment for infinite growth. The process is reversible, meaning that the cell with an infinite growth capacity obtained in the method of the invention is not a tumor cell.

The present invention exploits the fact that all the somatic cells of an individual contain the genetic information required to become any type of cell, and when placed in a conducive environment, a terminally differentiated cell's fate can be redirected to pluripotentiality. This fact has been exemplified by the success of somatic cell nuclear transfer experiments in nonhuman mammals. As normal development proceeds, the gene expression profile of a cell becomes restricted and regions of the genome are stably inactivated such that, under normal conditions, the cell cannot rejuvenate. The present invention provides a method inducing changes in nuclear function and consequently, changing the cell's identity. Somatic cells, such as granulosa cells, when treated with follicular fluid or components thereof, can be expanded indefinitely, but they will die when the follicular fluid is removed, even in the presence of serum-containing culture medium. In practising the present invention, no embryos or foetuses of any species are ever created or used and no mixing of human and non-human mitochondrial or genomic DNA ever occurs. All the methods of the invention can be performed *in vitro*. Rejuvenating/reprogramming follicular fluid is readily available in large quantities from local slaughterhouses.

As shown by the success of somatic cell nuclear transfer, the ability to erase the memory of an adult differentiated somatic cell and replace it with its long forgotten embryonic memory is limited only by the ability to manipulate the intra- and extra-cellular environment. By providing an adult cell with follicular fluid or components thereof, the present invention alters nuclear memory and induces nuclear changes that are commonly observed in pluripotent stem cells. Benefits and advantages of the invention include the following:

- Eliminating ethical problems (no need for human embryos or foetal tissue, embryos do not have to be used, created, or destroyed).
- No mitochondrial incompatibility (no nuclear transfer into an enucleated ooctye).

The method comprises the steps of providing a cell population, previously isolated from mature tissue, and de-differentiating the cells under expansion conditions. After being de-differentiated the cells can be induced to re-differentiate into a different somatic cell type. For that purpose the cells and/or tissues generated according to the invention can be re-differentiated in a second cell culture medium in the presence of at least one factor which induces and/or accelerates and/or promotes the re-differentiation of the cells.

Any of a variety of factors that increase differentiation of the cells can be used in the process of cell re-differentiation. Non-limiting examples of factors that may be used for re-differentiation are arachidonic acid, prostaglandin A, prostaglandin B, prostaglandin E, prostaglandin F, and histamine, with or without additional hormones/corticoids, like dexamethasone, and growth factors.

Those of ordinary skill in the art will appreciate the variety of cell types to which the described method of cell expansion and de-differentiation can be applied. Tissue engineering techniques have been used to correct defects by using a myriad of different cell types. Tissue engineering can be applied to the correction of hard tissue defects, such as defects in cartilage or bone that arise from disease or trauma. Tissue engineering has also been applied to the correction of soft tissue structures. By way of example, cells used in the current invention can be used to regenerate metabolic organs (the liver or pancreas), epidermal tissue (e.g. tissue of bum victims) or to reconstruct or augment breast tissue (e.g. muscle cells may be used to reconstruct the breast of women afflicted with breast cancer, congenital defects, or damage resulting from trauma; see U.S. Patent No. 5,512,600 and WO 96/18424,). Furthermore, congenital defects such as vesicoureteral reflux, or incontinence can be corrected by implantation of a gel or scaffolding matrix seeded with muscle cells in an effective amount to yield a muscle area that provides the required control over the passage of urine or otherwise corrects the defect (U.S. Patent No. 5,667,778).

Cells de-differentiated and cells re-differentiated as discussed above can be implanted with a suitable biodegradable, polymeric matrix to form new tissue. There are different forms of matrices which can be used. Non-limiting examples include a polymeric gel formed of a material such as alginate having cells suspended therein, fibrous matrices having an interstitial spacing between about 100 and 300 μm, and 3D foams. Matrices can be based on naturally occurring polymers (e.g. hyaluronic acid, collagen or the like) or synthetic polymers(e.g. poly-glycolic acid, poly-lactic acid or the like), or both. For a detailed description of hydrogel polymer solutions and polymeric matrices and other methods of implantation see US Patent 5,716,404. For other methods of using biodegradable polymers to regenerate metabolic organs and other tissues, see Cima et al., Biotech. Bioeng., 38, 145-158, 1991; Langer et al., Biomaterials, 11, 738-745, 1990; Vacanti et al., J. Pediatr. Surg., 23, 3-9,1988; and Vacanti et al., Arch. Surg., 123, 545-549, 1988.

In some embodiments, the cell matrix structures are implanted in combination with tissue expander devices.

As the cell matrix is implanted, or cells proliferate and form new tissue, the expander size is decreased, until it can be removed and the desired reconstruction or augmentation is obtained.

BRIEF DESCRIPTION OF THE DRAWINGS

The present invention will now be illustrated in more detail by the following examples, which are not meant to limit the scope of the invention. These examples are described with reference to the drawings.

Figure-1 shows the results of microscopic examination of granulosa cells after 8 days of incubation in a $CO_2$ incubator at 37°C in the presence of follicular fluid molecular size fractions (Table 12, Example J, Fractionation of follicular fluid). Numbers indicate number of well.

1: > 50 kDa; 2: 30-50 kDa, 3: 10-30 kDa; 4: 3-10 kDa: 5: < 3 kDa; 6: complete follicular fluid; 7: no follicular fluid; 8: > 50 and < 3 kDa; 9: Well coated with > 50 kDa; 10: Well coated with > 50 and < 3 kDa.

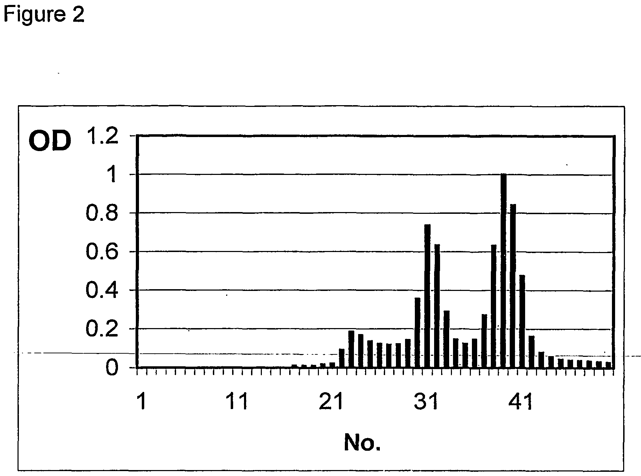

Figure 2 shows a graph of optical density (OD) at 280 nm (vertical axe) versus No. of collected fraction (horizontal axe) of a size chromatography of the fraction > 50 kDa from follicular fluid through Sephadex™ G200 super fine (Example K, Purification of the high molecular weight factor of follicular fluid).

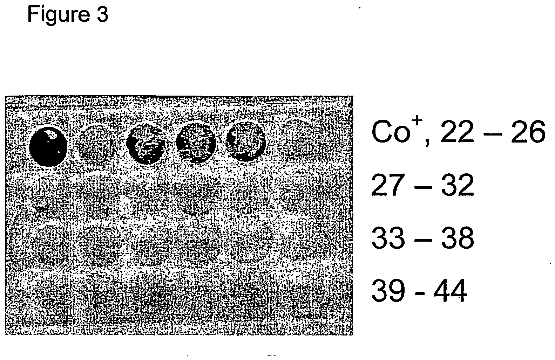

Figure 3 shows the results of a bioassay of the size chromatography fractions of Figure 2 in granulosa senescent cells (Example K, Purification of the high molecular weight factor of follicular fluid). $CO^+$: positive control. Only fraction 23, 24 and 25 contain active material.

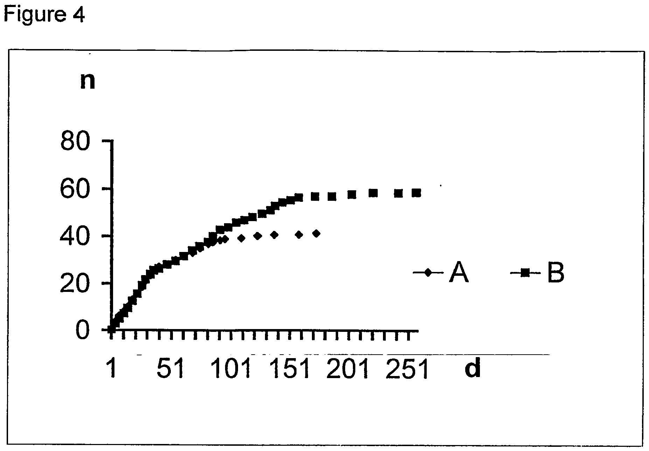

Figure 4 shows the number of population doublings (n) as a function of days in culture (d) of NHDF cells cultured in medium containing 10% foetal calf serum (A) or in medium containing 10% foetal calf serum and 4% follicular fluid (B). The proliferative lifespan of these cells is extended by about 15-20 population doublings by follicular fluid (Example G, Follicular fluid extends lifespan of primary human fibroblasts in culture).

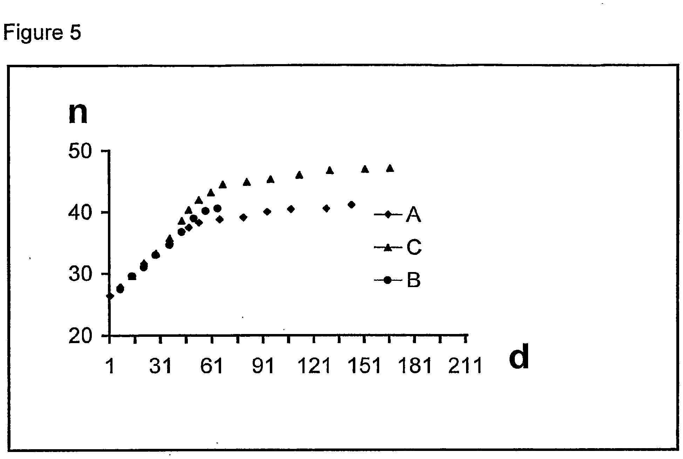

Figure 5 compares the number of population doublings (n) as a function of days in culture (d) of NHDF cells cultured in medium containing 10% foetal calf serum (A), in medium containing 10% foetal calf serum and 4% follicular fluid (B) and in medium containing 10% foetal calf serum and 4% charcoal filtered follicular fluid (C). Charcoal filtered follicular fluid is superior to untreated follicular fluid in delaying senescence (Example G, Follicular fluid extends lifespan of primary human fibroblasts in culture).

DESCRIPTION OF THE PREFERRED EMBODIMENTS

**A Preparation of Follicular Fluid**

*Collection of ovaries*

Ovaries are obtained from slaughtered pigs at least 4 months old in a local abattoir. Ovaries are collected from freshly sacrificed animals and placed immediately in sterile 0.9% NaCl (w/v) at a temperature of approximately 39°C in a thermobottle. They are transported to the laboratory within 2 h of recovery. The temperature of the container upon arrival is approximately 33°C.

*Collection of follicular fluid*

Ovaries are disinfected for 10-20 seconds in 70% ethanol prewarmed at 39°C, then dissected from fat and surrounding tissue, and put immediately into sterile phosphate buffer saline (PBS: 0.2 g/l KCl, 0.2 g/l $KH_2PO_4$, 8 g/l NaCl, 1.15 g/l $Na_2HPO_4$) equilibrated at 39°C on a thermoplate with a magnetic stirrer.

Follicular fluid is aspirated from peripheral antral follicles 3-8 mm in diameter in a 50 ml conical centrifuge tube (Orange Scientific, Belgium) using an aspiration pump under low vacuum and a 18-gauge needle. Cell aggregates, cumulus-oocyte-complexes (COC's) and cellular debris are sedimented at 39°C without applying centrifugal forces. The supernatant is collected and preserved frozen at -20°C until further processing. The pellet is used as a source of granulosa cells for cell culture.

*Preparation of follicular fluid for cell culture*

The follicular fluid preserved at -20°C is thawed and centrifuged at 1600 x g for 20 min at room temperature. The clear supernatant is removed and sterile filtered by passage through a 0.2 μm pore size filter (Sartorius, Germany, Cat. No 16534). The sterile follicular fluid is stored at 4°C or at -20°C.

*Preparation of charcoal filtered follicular fluid for cell culture*

1 g of activated charcoal (30-50 mesh ASTM, Merck Cat. No. 1.09631) is put into a Poly-Prep® column 10 ml (BioRad, Cat. No. 731-1550). The charcoal is washed sequentially with 10 ml of 0.2 N HCl, 10 ml of absolute ethanol, 10 ml of methanol, and 10 ml of 0.2 N NaOH. Before application of each new solution, the activated charcoal is thoroughly washed with distilled water.

Follicular fluid, clarified by centrifugation for 20 min, at 1600 x g, is passed four times through the charcoal column by gravity. The final flow-through is sterile filtered as above and stored at 4°C or at -20°C.

**B Culture of porcine granulosa cells**

Primary cell lines are established from granulosa cells collected by aspirating the ovarian antral follicles (3-8 mm in diameter) from slaughtered pigs at least one month old. The collected cells are sedimented without applying centrifugal force. The cell pellet at the bottom is the source of granulosa cells after manual removal of cumulus-oocyte complexes. The cell aggregates are washed once in PBS before resuspending them in granulosa cell medium. The cell suspension is dispensed into a tissue culture dish 15 cm in diameter (Orange Scientific, Belgium). Cultures are incubated at 39°C in a 5% $CO_2$ atmosphere. When the culture has reached confluency, the cells are passaged as indicated below.

Granulosa cell medium consists of the following components: 89% (v/v) DMEM (Dulbecco's Modified Eagle's medium, low glucose, BioConcept 1-25F50-I), 10% (v/v) FCS (foetal calf serum, BioConcept 1-01 F00-I), 0.1 mM NEAA (non-essential amino acids, BioConcept 5-13K00), 2.5 ng/ml bFGF (basic fibroblast growth factor, Sigma Product No. F 0291, 25 $\mu$g reconstituted in 1 ml granulosa cell medium lacking bFGF, frozen at -20°C until use), and 6 $\mu$g/ml gentamycin (BioConcept 4-07F00-H).

*Subcultures*

Culture dishes are washed twice with sterile PBS at room temperature. The cells are passaged using an enzymatic solution comprising 0.05% w/v trypsin (BioConcept, Switzerland) and 0.02% w/v ethylenedinitrilotetraacetic acid tetrasodium salt dihydrate (Sigma E-611) in PBS. After cell detachment the enzymatic activity is stopped by addition of FCS (2% final concentration). Cells are centrifuged for 5 minutes at room temperature at 180 x g. The cell pellet is resuspended in an appropriate volume of culture medium. An aliquot is taken for counting.

*Cell count*

An aliquot of the cell suspension is diluted in 0.05% trypan blue (BioConcept, Switzerland) in PBS and counted using a Neubauer hemocytometer. Live cells (excluding trypan blue dye) are counted.

*Cryopreservation of granulosa cells from cell culture*

Granulosa cells in culture are trypsinized and suspended in medium containing 72% DMEM low glucose (Bio-Concept, Switzerland), 20% FCS and 8% DMSO (dimethyl sulfoxide, Sigma). The cells are dispensed into cryogenic vials (1 ml) at a concentration between 0.5 - 3.0 x $10^6$ cells per ml. They are frozen in a controlled rate freezer (Nicool LM 10, France) adjusted to position 3 for 25 min (till-18°C), then to position 10 for 10 min (till -60°C). The cryogenic vials are then immediately transferred and stored in a liquid nitrogen tank.

**C Proliferative senescense of granulosa cells and rescue with follicular fluid**

*Establishment of a granulosa primary cell line*

800 cumulus-oocyte complexes are collected and suspended in 0.4 ml Tyrode's based solution containing 0.1-% (w/v) hyluronidase (Sigma product No. H-3884). The cumulus-oocyte complexes are disrupted by pipetting (1 ml tip) gently up and down for 1 minute at room temperature. Cells are pelleted by centrifugation (180 x g for 5 min) and resuspended in 2 ml Tyrode's based solution. Oocytes are removed mechanically with a pipette from the suspension under the microscope. Remaining cell suspension is mixed with 10 ml granulosa cell medium, put into a 10 cm in diameter plate, and incubated at 39°C in a 5% $CO_2$ incubator. Confluent monolayers are passaged, cells are counted at each passage, and the cumulative number of cells are recorded in Table 1.

Table 1: Granulosa primary cell line

| Days in culture | Passage number (subculture) | Cumulative number of cells |
|---|---|---|
| 8 | Primary culture | $2.1 \times 10^6$ |
| 12 | 1 | $3.2 \times 10^6$ |
| 18 | 2 | $5.7 \times 10^6$ |
| 22 | 3 | $28.8 \times 10^6$ |
| 39 | 4 | $34.2 \times 10^6$ |
| 54 | 5 | $8.2 \times 10^8$ |
| 74 | 6 | 0 |

In this representative experiment, the granulosa primary cell line has a finite lifespan entering proliferative senescence after 4 to 5 passages *in vitro.*

*Follicular fluid rescues from senescence*

$0.0557 \times 10^6$ granulosa cells are taken from passage 5 and cultured in 10 ml granulosa cell medium supplemented with follicular fluid (5% v/v), in a 10 cm in diameter culture dish. The rescuing effect of follicular fluid on senescent granulosa cells is shown in Table 2.

Table 2: Granulosa cells in the presence of follicular fluid

| Days in culture | Passage number (subculture) | Cumulative number of cells |
|---|---|---|
| 0 | 5 | $0.0557 \times 10^6$ |
| 24 | 6 | $0.55 \times 10^6$ |
| 31 | 7 | $6.8 \times 10^6$, |
| 37 | 8 | $43.53 \times 10^6$ |

The follicular fluid has rescued the proliferative senescent cells which then proliferate with a population doubling time of about 1.5 days. This granulosa cell line is still proliferating after 50 passages *in vitro* (more than 150 population doublings).

*Reversibility of the rescuing effect*

$1.07 \times 10^6$ granulosa cells are taken from passage 7 and cultured in 10 ml granulosa cell medium without follicular fluid or in 10 ml granulosa cell medium supplemented with follicular fluid (5% v/v), in 10 cm in diameter culture dishes. The reversibility of the effect of follicular fluid is shown in Table 3.

Table 3: Reversibility of the rescuing effect

| Days in culture | Passage number (subculture) | Cumulative number of cells (no follicular fluid supplement in medium) | Cumulative number of cells (5% v/v follicular fluid supplement in medium) |
|---|---|---|---|
| 0 | 7 | $1.07 \times 10^6$ | $1.07 \times 10^6$ |
| 6 | 8 | $1.95 \times 10^6$ | $6.68 \times 10^6$ |
| 11 | 9 | $< 0.2 \times 10^6$ | |

The rescuing effect of the follicular fluid is reversible since the cell line stops proliferating and cells die when the follicular fluid is removed. This means that the granulosa cell line has neither acquired properties of a tumor cell line nor has it been immortalised by a virus infection or the like.

*Characterization of granulosa cell lines established and maintained using follicular fluid.* Several features of cell lines derived from granulosa cells using the follicular fluid indicate that , although dividing rapidly, the cells have retained some characteristics of granulosa cells.

Morphology: Cells rich in microvilli or blebs of various sizes are observed in secondary cultures, in early passages (less than 10 passages in culture) and in late passages (more than 70 passages in culture). This feature has been described for granulosa cells (Motta P et al., 2003. Int. Rev. Cytology 223:178-288).

Steroid synthetic activity: Progression of granulosa cell differentiation is accompanied by production of progesterone (Motta P et al., loc. cit.). The production of progesterone by the granulosa cell lines is low (Table 4). It is likely that the cells have de-differentiated and are arrested at an early stage of cytodifferentiation.

Table 4: Production of progesterone by granulosa cell lines:

|  | Progesterone (pg/ml) | |
| --- | --- | --- |
|  | Experiment 1 | Experiment 2 |
| Medium, 0 h | 11.4 | 11.4 |
| Medium, 48 h | 64.3 | 34.2 |
| Medium + FSH, 48 h | 68.9 | 15.2 |

Apoptotic cell death: A rapid accumulation of pycnotic cells and a spontaneous onset of DNA fragmentation characteristic of apoptotic cell death occurs in granulosa cell lines, within 24 h after serum deprivation. Serum removal has been shown to accelerate the spontaneous onset of apoptotic cell death which occurs in primary cultures of granulosa cells (Hu C-L et al., 2001. Biol Reprod 64:518-526).

Contact inhibition: Like *in vivo,* granulosa cells from the established cell lines divide *in vitro* without contact inhibition. They form multilayers with cells in a criss-cross pattern.

## D Rescue of primary granulosa cell line with follicular fluid after cryo-preservation

Primary granulosa cell lines can be passaged for a finite number of times, then they enter a state of irreversibly arrested growth and death. Arrested growth is accelerated by cryo-preservation of granulosa cell lines. The following experiment shows that follicular fluid rescues granulosa cells after cryo-preservation and thawing.

A primary culture of pig granulosa cells is grown to confluency in a 15 cm in diameter culture dish. Cells are detached by trypsin/EDTA treatment, counted, and suspended at the concentration of $2.2 \times 10^6$ cells/ml of medium for cryo-preservation (see "B Culture of porcine granulosa cells").

Granulosa cells are then thawed and two aliquots of $0.6 \times 10^6$ cells are distributed into two 10 cm in diameter culture dishes containing 10 ml of regular medium for granulosa cells. One dish is supplemented with 10% (v/v) of follicular fluid. Both dishes are incubated at 37°C in a $CO_2$ incubator. After 7 days in cultures cells are counted and sub-passaged into new dishes, keeping the same culture conditions. Cells are counted after an additional period of 8 days in culture (Table 5).

Table 5: Rescue of cryo-preserved granulosa cells

| Days in culture after thawing | Passage number (subculture) | Cumulative number of cells (no follicular fluid supplement in medium) | Cumulative number of cells (10% follicular fluid supplement in medium) |
| --- | --- | --- | --- |
| 0 |  | $0.7 \times 10^6$ | $0.7 \times 10^6$ |
| 7 | 2 | $0.7 \times 10^6$ | $1.3 \times 10^6$ |
| 15 | 3 | $0.15 \times 10^6$ | $12.2 \times 10^6$ |

The follicular fluid added as supplement to cell culture medium allows the thawed granulosa cells to grow normally and prevents the arrested growth and death observed in granulosa cell culture not supplemented with follicular fluid.

## E Follicular fluid is not a substitute for serum

A primary granulosa cell line maintained for 11 passages in granulosa cell medium supplemented with 4% follicular fluid is subcultured into two 15 cm in diameter culture dishes. Each dish is seeded with $5 \times 10^6$ cells in 30 ml granulosa culture medium; one dish is supplemented with 4% (v/v) of follicular fluid. After 3 days of incubation at 37°C

in a $CO_2$ incubator, cells are trypsinized and counted.

No follicular fluid supplement in the culture medium: total of 7.4 x 10^6 cells corresponding to 0.2 population doublings/day. These cells are referred to as "senescent". 4% (v/v) follicular fluid supplement in the culture medium: total of 14.5 x 10^6 cells corresponding to 0.5 population doublings/day. These cells are referred to as "rescued".

Both types of cell populations ("senescent" and "rescued") are suspended in serum-free granulosa cell medium at a concentration of 0.04 x 10^6 cells/ml and distributed into wells of a 24-well plate (0.75 ml per well, 30'000 cells per well) or into wells of a 12-well plate (2 ml per well, 80'000 cells per well). After 90 min of attachment, the serum-free medium is removed, and replaced with 0.75 ml, and 2 ml, respectively, of medium composed as shown in Table 6.

Plates are incubated at 37°C in a $CO_2$ incubator for 7 days. The 12-well plate is used for counting cells, and the results are given in the table. The 24-well plate is used for staining with crystal violet, and the results for the "rescued" cells are shown in Table 6 below.

*Crystal violet staining of cell cultures*

The medium is aspirated, the cell layer washed once with PBS, and stained with 0.5% (w/v) crystal violet (in 30% (v/v) ethanol and 0.5% (v/v) glutaraldehyde) for about 10 min. After the crystal violet solution is decanted, the plate is rinsed with tap water and then dried in air.

Table 6: Granulosa cells grown in medium containing serum and/or follicle fluid

| Sample number | Growth medium composition | | | "rescued" granulosa cells | "senescent" granulosa cells |
|---|---|---|---|---|---|
| | FCS | bFGF | follicular fluid 4% | Cell count | Cell count |
| 1 | 10% | + | - | 10.6 x 10^4 | 1.1 x 10^4 |
| 2 | 2% | + | - | 2.6 x 10^4 | 0.15 x 10^4 |
| 3 | 0% | + | - | 1.45 x 10^4 | 0 |
| | | | | | |
| 4 | 10% | - | - | 7.8 x 10^4 | 0.2 x 10^4 |
| 5 | 2% | - | - | 4.35 x 10^4 | 0 |
| 6 | 0% | - | - | 0 | 0 |
| | | | | | |
| 7 | 10% | + | + | 92.6 x 10^4 | 89 x 10^4 |
| 8 | 2% | + | + | 14.7 x 10^4 | 12.2 x 10^4 |
| 9 | 0% | + | + | 0.2 x 10^4 | 1.55 x 10^4 |

FCS, preferably 10% (v/v), is necessary but not sufficient to prevent growth arrest and death of the primary granulosa cell line. Follicular fluid is necessary but not sufficient to prevent growth arrest and death of the primary granulosa cell line. Follicular fluid contains factor(s) which are necessary for the survival of the primary granulosa cell line and which are absent in FCS.

**F Effect of charcoal filtered follicular fluid on the growth and maintenance of granulosa cells**

The granulosa cell line is maintained in granulosa cell medium supplemented with 4% follicular fluid for 7 passages. 0.4 x 10^6 cells are taken from passage 7 and cultured in 10 ml granulosa cell medium supplemented either with 4% follicular fluid, or with 4% charcoal filtered follicular fluid, or in 10 ml granulosa cell medium deprived of follicular fluid, in 10 cm in diameter culture dishes. Each week cells are counted and sub-passaged into new dishes, keeping the same culture conditions. The cumulative number of cells are shown in Table 7.

Table 7: Granulosa cells in culture

| Passage no. (subculture) | Days in culture | Cumulative number of cells | | |
|---|---|---|---|---|
| | | 4% follicular fluid | 4% charcoal filtered follicular fluid | no follicular fluid |
| | | | | |
| 8 | 0 | $0.4 \times 10^6$ | $0.4 \times 10^6$ | $0.4 \times 10^6$ |
| 9 | 8 | $8.3 \times 10^6$ | $2.1 \times 10^6$ | $3 \times 10^6$ |
| 10 | 14 | $193 \times 10^6$ | $24.2 \times 10^6$ | 0 |
| 11 | 22 | $1'378 \times 10^6$ | 0 | |
| 12 | 26 | $31'010 \times 10^6$ | | |

The charcoal filtered follicular fluid is not capable to sustain the growth of granulosa cell lines for a long period of time

*A mixture containing follicular fluid and charcoal filtered follicular fluid is superior in biological activity to follicular fluid alone: Cryopreserved cells*

Cells from a primary culture of porcine granulosa cells are cryopreserved. They are used for comparing the effect of follicular fluid with that of the mixture consisting of one volume of follicular fluid and one volume of charcoal filtered follicular fluid. $1.3 \times 10^6$ thawed cells are cultured in 10 ml granulosa cell medium supplemented either with 4% follicular fluid, or with 4% of the follicular fluid 1:1 mixture described above, or in 10 ml granulosa cell medium without follicular fluid, in 10 cm in diameter culture dishes. At each passage cells are counted and seeded into new dishes, keeping the same culture conditions. The cumulative number of cells is shown in Table 8.

Table 8: Cryopreserved granulosa cells in culture with a 1:1 mixture of follicular fluid and charcoal filtered follicular fluid

| Passage no. (subculture) | Days in culture | Cumulative number of cells | | |
|---|---|---|---|---|
| | | 4% follicular fluid | 4% of a 1:1 mixture* | no follicular fluid |
| 1 | 0 | $1.3 \times 10^6$ | $1.3 \times 10^8$ | $1.3 \times 10^6$ |
| 2 | 10 | $0.41 \times 10^6$ | $0.38 \times 10^6$ | $0.34 \times 10^6$ |
| 3 | 21 | $0.51 \times 10^6$ | $0.29 \times 10^6$ | n.d. |
| 4 | 28 | $1.1 \times 10^6$ | $8.65 \times 10^6$ | n.d. |
| 5 | 35 | $9.35 \times 10^6$ | $84.3 \times 10^6$ | $0.04 \times 10^6$ ** |
| *1:1 mixture of follicular fluid and charcoal filtered follicular fluid<br>** passage no. 3 after 35 days. n.d. = not determined | | | | |

A majority of cryopreserved granulosa cells rapidly die after thawing, in all culture conditions tested. The 1:1 mixture of follicular fluid and charcoal filtered follicular fluid is superior to follicular fluid alone in rescuing granulosa cells in culture, which otherwise die when they are deprived of follicular fluid. Rescue from senescence occurs earlier.

*A mixture containing follicular fluid and charcoal filtered follicular fluid is superior in biological activity to follicular fluid alone: Proliferation and growth of granulosa cell lines*

The granulosa cell line is maintained in granulosa cell medium supplemented with 4% follicular fluid for 8 passages. $0.4 \times 10^6$ cells are taken from passage 8 and cultured in 10 ml granulosa cell medium supplemented either with 4% follicular fluid, or with 4% of the 1:1 mixture of follicular fluid and charcoal filtered follicular fluid, or in 10 ml granulosa cell medium deprived of follicular fluid, in 10 cm in diameter culture dishes. When cultures are confluent, cells are counted and sub-passaged into new dishes, keeping the same culture conditions. The cumulative number of cells are shown in Table 9:

Table 9: Granulosa cell line in culture with a 1:1 mixture of follicular fluid and charcoal filtered follicular fluid

| Passage no. (subculture) | Days in culture | Cumulative number of cells | | |
|---|---|---|---|---|
| | | 4% follicular fluid | 4% of a 1:1 mixture* | no follicular fluid |
| 9 | 0 | $0.4 \times 10^6$ | $0.4 \times 10^6$ | $0.4 \times 10^6$ |
| 10 | 6 | $5.6 \times 10^6$ | $7 \times 10^6$ | $0.29 \times 10^6$ |
| 11 | 11 | $57.4 \times 10^6$ | $90.3 \times 10^6$ | 0 |
| 12 | 17 | $723.2 \times 10^6$ | $1463 \times 10^6$ | |
| 13 | 21 | $15.09 \times 10^9$ | $26.8 \times 10^9$ | |
| 14 | 27 | $379.2 \times 10^9$ | $850.9 \times 10^9$ | |
| 15 | 31 | $4.1 \times 10^{12}$ | $17 \times 10^{12}$ | |
| *1:1 mixture of follicular fluid and charcoal filtered follicular fluid | | | | |

The results show that granulosa cell lines proliferate faster in medium supplemented with the 1:1 mixture of follicular fluid and charcoal filtered follicular fluid than in medium containing follicular fluid alone. In this experiment, there is an average increase of 25% in the number of cells at each passage.

## G Follicular fluid extends lifespan of primary human fibroblasts in culture

Normal somatic cells invariably enter a state of irreversibly arrested growth and altered function after a finite number of divisions. This process is termed replicative senescence. Fibroblasts display a limited *in vitro* lifetime which is determined by the number of population doublings (PDs) rather than by the chronologic time. After explantation the cells undergo an initial period of rapid division which is followed by the slowing down of the proliferative activity (pre-senescent state). Subsequently the cells reach a state of senescence characterized by morphologic alterations such as increase in size and irregular shape, and they remain in a viable, though non-proliferative state for many months (Cristofalo VJ and Pignolo RJ, 1993. Physiol. Reviews 73:617-638).
Culture medium supplemented with follicular fluid delays the onset of senescence and allows cells to keep dividing over 15-20 PDs, which represents a 50% increase above normal.

*Senescence-associated beta-galactosidase staining of in normal human dermal fibroblast (NHDF) cells with or without addition of follicular fluid*

Beta-galactosidase, histochemically detectable at pH 6, is a biomarker that identifies senescent human fibroblasts in culture (Dimri et al., 1995. Proc. Nat. Acad. Sci. 92:9363-9367). Normal human dermal fibroblasts (NHDF, Cat. No. C-10351, PromoCell GmbH, Heidelberg, Germany) derived from foreskin are grown in PromoCell Fibroblast growth medium (Cat. No. C-23010). After adding PromoCell Supplement Mix/Fibroblast growth medium (Cat. No. C-39315), the concentration of growth factors in the complete medium are as follows: Insulin 5 $\mu$g/ml, basic fibroblast factor 1 ng/ml, amphotericin B 50 ng/ml, gentamicin 50 $\mu$g/ml. The PromoCell Fibroblast growth medium is a sterile liquid culture medium for culturing fibroblasts and is serum-free. Feeder-layer, matrix substrates or other substances are not necessary. Culturing and subculturing into a new culture container are performed according to the indications of the manufacturer.
13'500 NHDF cells after five passages using the PromoCell Fibroblast growth medium are seeded in one well of a 24-well plate in 0.75 ml of medium. 13'500 NHDF cells after six passages using the PromoCell Fibroblast growth medium supplemented with 4% (v/v) follicular fluid are seeded in another well of the 24-well plate in 0.75 ml medium supplemented with follicular fluid. After 3 days of incubation in a $CO_2$ incubator at 37°C, the cells are fixed and stained for senescence associated $\beta$-gatactosidase activity.

*Senescence associated $\beta$-galactosidase staining of cells*

The medium is aspirated and the cells washed once with PBS, then treated with 0.5% glutaraldehyde (25% solution, Sigma) in PBS at room temperature. After 5 minutes the cells are washed with 1 mM $MgCl_2$ in PBS, pH 7.2. Cells are stained in a solution containing 1 mg/ml X-gal (from 4% DMSO stock solution, Fermentas No. R04019), 0.12 mM $K_3Fe(CN)_6 3H_2O$, 0.12 mM $K_4Fe(CN)_6$, 1 mM $MgCl_2$ in PBS pH 6.0 (PBS adjusted to pH 6.0 by addition of acetic acid). The cells are incubated at room temperature or 37°C, washed once with $H_2O$, and stored in $H_2O$ at 4°C.

All fibroblasts maintained in the absence of follicular fluid are in a pre-senescent or senescent state. Less than 10% of fibroblasts maintained in medium supplement with follicular fluid are in a pre-senescent or senescent state.

*Culturing of normal human dermal fibroblasts*

Normal human dermal fibroblasts (NHDF, Cat.-No.: C-10351, PromoCell GmbH, Heidelberg, Germany) derived from foreskin are cultured in NHDF medium (89% (v/v) DMEM, 10% (v/v) FCS, 0.1 mM NEAA, and 6 $\mu$g/ml gentamycin). Culture dishes are washed twice with sterile PBS at room temperature. The cells are passaged using an enzymatic solution comprising 0.05% w/v trypsin and 0.02% w/v ethylenedinitrilo tetraacetic acid tetrasodium salt dihydrate in PBS. After cell detachment, the enzymatic activity is stopped by addition of FCS (2% final concentration). Cells are centrifuged for 5 minutes at room temperature at 180 x g. The cell pellet is resuspended in an appropriate volume of culture medium. An aliquot is taken for counting.

Cells are counted and cryopreserved as described for granulosa cells (B Culture of porcine granulosa cells).

*Follicular fluid expands the lifespan of NHDF*

The number of population doublings achieved by the culture at each passage is calculated from the formula

$$\text{population doublings} = (\log N_t - \log N_0)/\log 2.0,$$

where $N_t$ = the number of cells recovered at harvesting at time $t$, and $N_o$ = the number of cells originally plated.

The population doublings (PDs) of NHDF cells cultured in medium containing 10% serum or in medium containing 10% serum and 4% follicular fluid have been compared. The results are shown in Figure 4. The proliferative lifespan of these cells is extended by about 15-20 population doublings (50% increase in population doublings over the normal) as compared with that of their counterparts. Similar results are obtained with 5% serum concentration in the culture medium instead of 10%.

*Reversibility of the effect of follicular fluid*

0.45 x 10^6 NHDF cells grown continuously in NHDF medium containing 4% follicular fluid are taken at passage 21 (35 population doublings) and cultured in 10 ml NHDF medium without follicular fluid or in 10 ml NHDF medium supplemented with follicular fluid (4% v/v), or in 10 ml culture medium supplemented with charcoal filtered follicular fluid (4% v/v), in 10 cm in diameter culture dishes. The effect of follicular fluid is reversible as shown in Table 10.

Table 10: NHDF cells cultured in NHDF medium with or without follicular fluid

| Days in culture | Passage no. (subculture) | Cumulative number of cells | | |
|---|---|---|---|---|
| | | no follicular fluid | 4% follicular fluid | 4% charcoal filtered follicular fluid |
| 0 | 21 | 0.65 x 10^6 | 0.65 x 10^6 | 0.65 x 10^6 |
| 7 | 22 | 3.04 x 10^6 | 2.85 x 10^6 | 4.12 x 10^6 |
| 14 | 23 | 3.46 x 10^6 | 6.78 x 10^6 | 9.52 x 16^6 |
| 21 | 24 | 3.6 x 10^6 | 9.02 x 10^6 | 15.9 x 10^6 |

Withdrawal of follicular fluid from NHDF continuously grown in its presence renders the cells senescent. Charcoal filtered follicular fluid is superior to untreated follicular fluid in preventing senescence of NHDF cells.

*The rejuvenating effect of follicular fluid on pre-senescent NHDF cells*

Follicular fluid is capable, after addition to aged NHDF cell cultures reaching the pre-senescent state, of restoring their growth capacity.

NHDF cells passaged for 26 PD's in the absence of follicular fluid are further passaged in NHDF medium containing 4% follicular fluid as supplement. Likewise, NHDF cells passaged for 28 PD's in the absence of follicular fluid are further passaged in medium containing 4% charcoal filtered follicular fluid. NHDF cells are counted at each passage.

Results are shown in Figure 5.

Senescence is delayed by addition of follicular fluid. Charcoal filtered follicular fluid is superior to untreated follicular fluid in eliciting this effect.

**H Follicular fluid protects brain neurons in primary culture from cell death.**

*Preparation of primary neurons from embryonic rat brain cortex*

Cortices from embryonic (18 day) rat brain are dissected and treated with a papain solution for a total of 30 min. After addition of trypsin inhibitor, the cortical neurons are dissociated by gentle trituration with a pipette. The primary neurons are then counted and plated on standard tissue culture plates coated with poly-D lysine.

*Culturing of primary neurons*

After 2 hours of plating of primary neurons, the plating medium is replaced by the growth medium consisting of 5% FCS/DMEM (high glucose 4500 mg/ml) with or without 4% follicular fluid. Primary neuron cultures are maintained at 37°C in a $CO_2$ incubator for 24 to 48 hrs and examined using a light microscope.

Primary neuron cultures cultured in the absence of follicular fluid undergo extensive cell death with little or no neurite extension. Primary neuron cultures maintained in the presence of follicular fluid show survival of neurons with extensive neurite outgrowth, already after 24 hrs of incubation.

Follicular fluid provides protective action against neural cell death; rescues primary neuron cells from degeneration in *in vitro* conditions, and promotes outgrowth of neurites as well as allows for extensive formation of neural networks.

**I Follicular fluid components protect hepatocytes in primary culture from cell death.**

*Preparation of hepatocytes from embryonic rat liver*

Livers from rat embryos (18 day) are dissected and the hepatocytes are dissociated by gentle trituration with a pipette. The primary hepatocytes are plated on standard tissue culture plates. The growth medium consists of 5% FCS/DMEM with or without 4% charcoal filtered follicular fluid or 4% of the 1:1 mixture of follicular fluid and charcoal filtered follicular fluid.

*Culturing of primary hepatocytes*

Primary hepatocyte cultures are maintained at 37°C in a $CO_2$ incubator and examined using a light microscope. When the monolayer is confluent the cells are passaged using an enzymatic solution comprising 0.05% w/v trypsin (BioConcept, Switzerland) and 0.02% w/v ethylenedinitrilo tetraacetic acid tetrasodium salt dihydrate (Sigma E-611) in PBS. After cell detachment the enzymatic activity is stopped by addition of FCS (2% final concentration). Cells are centrifuged for 5 minutes at room temperature at 180 x g. The cell pellet is resuspended in an appropriate volume of culture medium. An aliquot is taken for counting. The effect of follicular fluids components is shown in Table 11.

Table 11: Embryonic rat hepatocytes in culture with a 1:1 mixture of follicular fluid and charcoal filtered follicular fluid or with charcoal filtered follicular fluid.

| Days in culture | Cumulative number of cells | | |
|---|---|---|---|
| | 4% of a 1:1 mixture* | 4% charcoal filtered follicular fluid | No follicular fluid |
| 0 | not counted | not counted | not counted |
| 10 | $14 \times 10^3$ | $10.3 \times 10^3$ | $5 \times 10^3$ |
| 17 | $90 \times 10^3$ | | |
| 20 | | | all cells are dead |
| 33 | $85 \times 10^3$ | $< 1 \times 10^3$ | |
| *1:1 mixture of follicular fluid and charcoal filtered follicular fluid | | | |

Primary embryonic hepatocyte cultures cultured in the absence of follicular fluid components do not survive for a prolonged period of time. The 1:1 mixture of follicular fluid and charcoal filtered follicular fluid provides protective action against hepatocyte cell death and rescues primary hepatocytes from degeneration in *in vitro* conditions.

## J Fractionation of follicular fluid

CENTRIPREP® Centrifugal Filter Devices YM-3 (nominal molecular weight limit 3'000), YM-10 (MW 10'000), YM-30 (MW 30'000), and YM-50 (MW 50'000) (Millipore Corporation, Product nos. 4320, 4321, 4322, and 4323, respectively) are used as ultrafiltration devices for purifying, concentrating, and desalting biological samples. The filtration process itself is gentle, avoiding potential problems such as sample denaturation and concentration of buffer salts. 32 ml of clarified follicular fluid are centrifuged at 1500 x g in a swinging-bucket rotor at room temperature. Concentration and dialysis with PBS of molecular size fractions is done according to the indications of the manufacturer. Starting with the CENTRIPREP® YM-50 device, the follicular fluid is sequentially fractionated and concentrated.

### Bioactivity of fractions from follicular fluid

$0.03 \times 10^6$ senescent granulosa cells (Passage 11) in 0.75 ml of granulosa cell medium are seeded per well of a 24-well plate. A volume calculated to restore the original 4% v/v follicular fluid is added to individual wells. One well receives no supplement (negative control). One well receives 30 µl of plain follicular fluid (positive control).

The results of microscopic examination after 8 days of incubation in a $CO_2$ incubator at 37°C are given in Table 12:

Table 12: Activity of follicular fluid molecular weight fractions in rescuing granulosa cells

| Fraction | Cell growth |
|---|---|
|  |  |
| > 50 kDa | ++++ |
| 30-50 kDa | - |
| 10-30 kDa | - |
| 3-10 kDa | - |
| < 3 kDa | ++ |
| Follicular fluid | +++++ |
| No addition | - |

The < 3 kDa fraction and the > 50 kDa fractions contain active factors which partially mimic the activity of the follicular fluid.

### Test for synergy between fractions < 3 kDa and > 50 kDa, and activity of fraction > 50 kDa after coating

$0.03 \times 10^6$ senescent granulosa cells (passage 16, deprived of follicular fluid during passage 15) in 0.75 ml of granulosa cell medium containing are seeded per well of a 24-well plate. A volume (calculated to restore the original 4% v/v of follicular fluid of each fraction) is added to individual wells. One well receives no supplement (negative control). One well receives 30 µl of plain follicular fluid (positive control). Coating is done in the following way: 350 µl of a 1% (v/v) solution of concentrated fraction > 50 kD in PBS is added to an empty well to be coated, and incubated at 37°C for one hour. After a rapid wash with water, the well is dried.

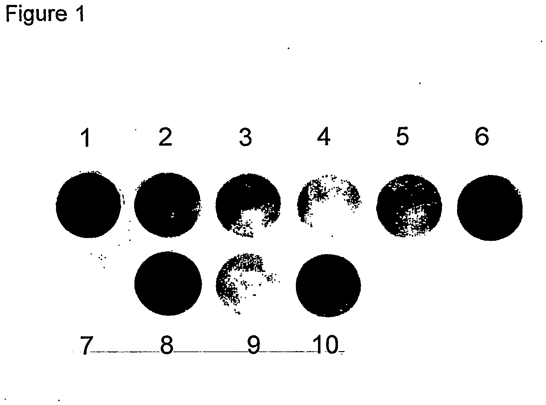

The results of microscopic examination after 8 days of incubation in a $CO_2$ incubator at 37°C are given in the Table 13 and shown in Figure 1:

Table 13: Activity of follicular fluid molecular weight fractions in supporting granulosa cell growth

| Well no. | Fraction | Cell growth |
|---|---|---|
|  |  |  |
| 1 | > 50 kDa | ++++ |
| 2 | 30-50 kDa | ++ |

(continued)

| Well no. | Fraction | Cell growth |
|---|---|---|
| 3 | 10-30 kDa | ++ |
| 4 | 3-10 kDa | + |
| 5 | < 3 kDa | ++ |
| 6 | Follicular fluid | +++++ |
| 7 | No addition | - |
| 8 | > 50 kDa + < 3 kDa | ++++++ |
| 9 | Well coated with fraction > 50 kDa | + |
| 10 | Well coated with fraction > 50 kDa + < 3 kDa | +++ |

**K Purification of the high molecular weight factor of follicular fluid**

*Size chromatography*

0.5 ml of fraction > 50 kDa from follicular fluid (protein concentration approx. 100 mg/ml) is chromatographed by gel filtration through Sephadex™ G200 super fine in a column 1 m in length and 26 mm in diameter using PBS buffer at 120 drops / fraction (corresponding to 6-7 ml).
Figure 2 shows a graph of the optical density at 280 nm (vertical axis) of collected fractions (horizontal axis). Three main peaks are separated. The fractions are tested for biological activity and protein properties as follows:

*Bioassay*

0.02 x $10^6$ granulosa senescent cells (Passage 30) in 750 $\mu$l granulosa culture medium are seeded per well in a 24-well plate. Six hours after plating, 350 $\mu$l of medium are removed and replaced by 200 $\mu$l of the mixture composed of 100 $\mu$l of the fraction to be tested and 100 $\mu$l of DMEM containing 20% FCS, 0.2 mM NEAA and 12 $\mu$g/ml gentamycin. Positive control: in the first well, 350 $\mu$l of medium are removed and replaced by 230 $\mu$l of the mixture composed of 100 $\mu$l of PBS, 100 $\mu$l of DMEM containing 20% FCS, 0.2 mM NEAA and 12 $\mu$g/ml gentamycin and 30 $\mu$l of follicular fluid. After four days of incubation at 37°C in a $CO_2$ incubator, the plate is stained with crystal violet.
Results are shown in Figure 3. In this figure the upper left well is a positive control. Next wells on the right are fractions 22 to 44.
Activity for rescuing granulosa cells is detected in fractions 23, 24 and 25. The activity corresponds to the peak of highest size in the chromatography.

*Protein analysis by sodium dodecyl sulfate (SDS) polyacrylamide gel electrophoresis PAGE*

Analysis was performed using the NuPAGE® Novex Bis-Tris Gel system according to the instructions of the manufacturer on a XCell *SureLock™* Mini-Cell for electrophoresis of mini-gels (Invitrogen Cat. Nos: EI0001, EI0020, EI0002), using pre-cast polyacrylamide gel 4-12% Bis-Tris Gel, 1.0 mm x 12 well (Invitrogen Cat No. NP0322) and NuPAGE® MES SDS running buffer (Invitrogen Cat. No. NP0002).
5 $\mu$l NuPAGE® LDS Sample buffer (4X) and optionally 2 $\mu$l NuPAGE® Reducing Agent (10X) are added to 15 $\mu$l samples of fractions to be analysed, resulting in a total volume of 20 $\mu$l and 22 $\mu$l, respectively. Samples are heated at 70°C for 10 minutes. After electrophoresis, the gel is stained with a solution of 0.125% (w/v) Coomassie Blue (Coomassie® Brilliant Blue R250, Fluka Cat. No. 27816), 50% methanol and 10% acetic acid for 15 minutes on a shaker. The gel is destained in 5% methanol and 7% acetic acid.
The major protein component of fractions 23-25 has an apparent MW of » 220 kDa under denaturing, non-reducing conditions, and of approx.170 kDa under denaturing and reducing conditions. It is likely to be composed of a dimer of two 160 kDa subunits linked by disulfide bridge(s). Proteins in fractions 30-32 and in fractions 38-41 correspond to immunoglobulins and albumin, respectively.

*Cation exchange chromatograph*

The pool of fractions 23-25 from the size chromatography is concentrated on CENTRIPREP® and dialysed against 25 mM acetate buffer pH 5.0 containing 0.02% $NaN_3$. chromatography is performed with CM cellulose ion exchanger on a bed volume of 0.5 ml using a start buffer of 25 mM acetate pH 5.0 containing 0.02% $NaN_3$. Fractions collected are shown in Table 14.

Table 14: Cation exchange chromatography fractions

| Sample | OD 280 nm (1 cm) | Fraction no. | Activity |
|---|---|---|---|
| | | | |
| Start buffer | 0.092 | | |
| Sample "flow through" (1.05 ml) | 0.577 | 1 | + |
| First wash with 1.05 ml start buffer | 0.142 | 2 | not tested |
| Second wash with 1.05 ml start buffer | 0.127 | 3 | - |
| Third wash with 1.05 ml start buffer | 0.109 | 4 | not tested |
| Elution with 1.05 ml 500 mM NaCl in 25 mM acetate buffer | 0.113 | 5 | - |
| 500 mM NaCl in 25 mM acetate buffer | 0.095 | | |

Bioassay: Since the samples contain 0.02% $NaN_3$ (toxic for living cells), the activity is tested after coating of the components to wells. 100 $\mu$l of samples from the CM column (fractions number 1, 3 and 5) are mixed with 100 $\mu$l of PBS and the total volume is transferred into a well of a 24-well plate. The plate is incubated for two hours at 37°C, and then overnight at 4°C. The sample is removed. The well is washed with sterile water and dried.

$0.02 \times 10^6$ senescent granulosa cells are seeded to each well in 0.75 ml medium for granulosa cells. Positive control: no coating, addition of 30 $\mu$l of follicular fluid to the culture medium. Negative control: no coating, no addition. After four days of incubation at 37°C in a $CO_2$ incubator, the plate is stained with crystal violet.

The bulk of proteins as well the active factor are in the "flow through" fraction of the cation exchange chromatography.

_Protein identification by peptide mass fingerpinting_

Aliquots of the "flow through" fraction are separated by polyacrylamide gel electrophoresis, one sample denatured in reducing conditions and one sample denatured in non-reducing conditions.

Gels are stained for 3 minutes at room temperature on a shaker in 0.15% (w/v) of Coomassie Blue G-250, 0.5% (v/v) acetic acid and 10% methanol in high-purity water. The gel is then destained at room temperature on a shaker in 0.5% (v/v) acetic acid and 10% (v/v) methanol in high-purity water until protein bands are visible.

The band with relative MW > 220 kDa under non-reducing conditions, and the band 160 kDa under reducing conditions are cut and transferred to separate 0.5 ml safe-lock tubes. 200 $\mu$l high-purity water are added and the samples are frozen at -20°C until use for mass spectrometry.

Eight peptides are detected which correspond to amino acid sequences of the human alpha-2-macroglobulin precursor [A2MG_human; P01023; mass (average) 163278] and to homologs of other species (guinea pig and mouse). The pig protein is not in the SwissProt/Trembl data base. The sample contains a new pig protein which belongs to the family of the alpha-2-macroglobulin precursor.

Peptides detected are:

1) TEVSSNHVLIYLDK
2) QQNAQGGFSSTQDTVVALHALSK
3) MVSGFIPLKPTVK
4) SSGSLLNNAIK
5) QTVSWAVTPK
6) GEAFTLK
7) YGAATFTR
8) DLKPAIVK

The members of this family of proteins are in the form of a homotetramer, which consists of two pairs of disulfide-linked chains. This property fits with the SDS-PAGE analysis of the pig protein.

**L Karyotyping**

*Preparation of mitotic spreads*

Colcemid® (Fluka Cat. No. 27645, stock solution 5 μg/ml in distilled water, stored at -20°C) is added to a final concentration of 0.05 μg/ml to a 6 cm plate of growing cells. The cells are incubated at 37°C for 2.5 h. The cells are trypsinized and resuspend in 10 ml of 0.56% (w/v) KCl solution. The cells are left at room temperature for a total of 20 min (including the centrifugation time below). The cells are pelleted by gentle centrifugation (500 rpm, 5 min). As much as possible of the KCl solution is aspirated. 1 ml of Carnoy's fixative (3:1 v/v absolute methanol / glacial acetic acid) is added. After 5 min at room temperature, the cells are pelleted and the fixative changed. This is repeated once, and the cells suspended in 1 ml Carnoy's fixative. Small single drops of the cell suspension are applied onto precleaned glass microscope slides. The slides are stained for 30 min with 300 nM DAPI (4',6-diamidino-2-phenylindole, Sigma-Aldrich) stock solution in PBS, then washed in PBS. Pictures are taken using a florescence microscope, and the chromosomes counted on the pictures.

Cells maintained for prolonged periods of time in follicular fluid have a normal complement of chromosomes. The results of counting several mitotic spreads in each preparation are shown in Table 15.

Table 15: Chromosome counting

| Cells | History | Counted | Expected |
|---|---|---|---|
| | | | |
| Granulosa cell line A (pig) | 70 Passages (more than 200 population doublings) in medium containing 4% follicular fluid | 38 | 2N = 38 |
| Granulosa cell line B (pig) | 21 Passages (more than 60 population doublings) in medium containing 4% follicular fluid | 38 | 2N = 38 |
| NHDF cells (human) | 50 population doublings in medium containing 4% follicular fluid | 46 | 2N = 46 |

**Claims**

1. A method for preventing senescence and death of somatic cells comprising culturing the cells in a culture medium containing follicular fluid or components thereof obtained by fractionation according to molecular mass and/or polarity.

2. The method according to claim 1 comprising the steps of

   - culturing the cells in a serum-containing medium;
   - introducing a follicular fluid or components thereof into the culture medium and allowing indefinite proliferation of cells in repeated subcultures.

3. The method according to claim 1 wherein the follicular fluid is partially purified.

4. The method according to claim 3 wherein the follicular fluid is charcoal filtered.

5. The method according to claim 3 wherein the follicular fluid is an approximately 1: 1 mixture of unfiltered follicular fluid and charcoal filtered follicular fluid.

6. The method according to claim 1 wherein the components of follicular fluid are the molecular size fractions > 30 kDa.

7. The method according to claim 1 wherein the components of follicular fluid are the combined molecular size fractions > 50 kDa and < 3 kDa.

8. The method according to claim 1 wherein the components of follicular fluid are peptides with an amino acid sequences of the human alpha-2-macroglobulin precursor or of homologs of other species.

9. The method according to claim 1 wherein the follicular fluid is selected from the group of porcine, bovine, ovine and equine follicular fluid.

10. The method according to claim 9 wherein the follicular fluid is porcine follicular fluid.

11. The method according to claim 1 wherein the cells are granulosa cells, dermal fibroblasts cells, neurons, keratinocytes or hepatocytes.

12. The method according to claim 11 wherein the cells are granulosa cells, dermal fibroblast cells, neurons or hepatocytes.

13. Use of a follicular fluid or components thereof for preventing senescence and death of somatic cells by in vitro culturing the cells in a medium containing follicular fluid or components thereof obtained by fractionation according to molecular mass and/or polarity.

**Patentansprüche**

1. Verfahren zum Verhindern des Alterns und Tods von Körperzellen, das das Kultivieren der Zellen in einem Nährmedium umfasst, das Follikularflüssigkeit oder Bestandteile davon enthält, die durch Fraktionierung nach Molekülmasse und/oder Polarität erhalten werden.

2. Verfahren nach Anspruch 1, das folgende Schritte umfasst:

   - Kultivieren der Zellen in einem serumhaltigen Medium,
   - Einbringen einer Follikularflüssigkeit oder von Bestandteilen davon in das Nährmedium und
   - Ermöglichen einer unbegrenzten Zellproliferation in mehreren Subkulturen.

3. Verfahren nach Anspruch 1, wobei die Follikularflüssigkeit teilweise gereinigt wird.

4. Verfahren nach Anspruch 3, wobei die Follikularflüssigkeit über Kohle gefiltert wird.

5. Verfahren nach Anspruch 3, wobei die Follikularflüssigkeit eine ungefähre 1:1-Mischung aus ungefilterter Follikularflüssigkeit und über Kohle gefilterter Follikularflüssigkeit ist.

6. Verfahren nach Anspruch 1, wobei die Bestandteile der Follikularflüssigkeit die Fraktionen mit einer Molekülgröße von > 30 kDa sind.

7. Verfahren nach Anspruch 1, wobei die Bestandteile der Follikularflüssigkeit die kombinierten Fraktionen mit einer Molekülgröße von > 50 kDa und < 3 kDa sind.

8. Verfahren nach Anspruch 1, wobei die Bestandteile der Follikularflüssigkeit Peptide mit einer Aminosäurensequenz des Präkursors von Alpha-2-Makroglobulin vom Menschen oder von Homologen anderer Arten sind.

9. Verfahren nach Anspruch 1, wobei die Follikularflüssigkeit aus der Gruppe der Follikularflüssigkeit vom Schwein, Rind, Schaf und Pferd ausgewählt ist.

10. Verfahren nach Anspruch 9, wobei die Follikularflüssigkeit Follikularflüssigkeit vom Schwein ist.

11. Verfahren nach Anspruch 1, wobei die Zellen Granulosazellen, Hautfibroblasten, Neuronen, Keratinozyten oder Hepatozyten sind.

12. Verfahren nach Anspruch 11, wobei die Zellen Granulosazellen, Hautfibroblasten, Neuronen oder Hepatozyten sind.

13. Verwendung einer Follikularflüssigkeit oder von Bestandteilen davon zum Verhindern des Alterns und Tods von Körperzellen durch in-vitro-Kultivierung der Zellen in einem Medium, das Follikularflüssigkeit oder Bestandteile davon enthält, die durch Fraktionierung nach Molekülmasse und/oder Polarität erhalten werden.

**Revendications**

1. Procédé de prévention de la sénescence et de la mort de cellules somatiques, comprenant la culture des cellules dans un milieu de culture contenant un fluide folliculaire ou des composants de celui-ci obtenus par fractionnement selon la masse moléculaire et/ou la polarité.

2. Procédé selon la revendication 1, comprenant les étapes consistant à :

   - cultiver les cellules dans un milieu contenant du sérum ;
   - introduire un fluide folliculaire ou des composants de celui-ci dans le milieu de culture ; et

- permettre une prolifération indéfinie de cellules en sous-cultures répétées.

3. Procédé selon la revendication 1, dans lequel le fluide folliculaire est partiellement purifié.

4. Procédé selon la revendication 3, dans lequel le fluide folliculaire est filtré sur charbon de bois.

5. Procédé selon la revendication 3, dans lequel le fluide folliculaire est un mélange approximativement 1:1 de fluide folliculaire non filtré et de fluide folliculaire filtré sur charbon de bois.

6. Procédé selon la revendication 1, dans lequel les composants de fluide folliculaire sont les fractions de dimension moléculaire > 30 kDa.

7. Procédé selon la revendication 1, dans lequel les composants de fluide folliculaire sont les fractions de dimension moléculaire combinées > 50 kDa et < 3 kDa.

8. Procédé selon la revendication 1, dans lequel les composants de fluide folliculaire sont des peptides ayant des séquences d'acides aminés du précurseur de l'alpha-2-macroglobuline humaine ou d'homologues d'autres espèces.

9. Procédé selon la revendication 1, dans lequel le fluide folliculaire est choisi dans le groupe de fluide folliculaire porcin, bovin, ovin et équin.

10. Procédé selon la revendication 9, dans lequel le fluide folliculaire est le fluide folliculaire porcin.

11. Procédé selon la revendication 1, dans lequel les cellules sont des cellules de la granulosa, des cellules fibroblastes dermiques, des neurones, des kératinocytes ou des hépatocytes.

12. Procédé selon la revendication 11, dans lequel les cellules sont des cellules de la granulosa, des cellules fibroblastes dermiques, des neurones ou des hépatocytes.

13. Utilisation d'un fluide folliculaire ou de composants de celui-ci pour prévenir la sénescence et la mort de cellules somatiques par la culture *in vitro* des cellules dans un milieu contenant un fluide folliculaire ou des composants de celui-ci obtenus par fractionnement selon la masse moléculaire et/ou la polarité.

Figure 1

Figure 2

Figure 3

Co⁺, 22 – 26

27 – 32

33 – 38

39 - 44

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6323025 B
- US 5512600 A
- WO 9618424 A
- US 5667778 A
- US 5716404 A
- EP 03405628 A

### Non-patent literature cited in the description

- **Cima et al.** *Biotech. Bioeng.,* 1991, vol. 38, 145-158
- **Langer et al.** *Biomaterials,* 1990, vol. 11, 738-745
- **Vacanti et al.** *J. Pediatr. Surg.,* 1988, vol. 23, 3-9
- **Vacanti et al.** *Arch. Surg.,* 1988, vol. 123, 545-549
- **Motta P et al.** *Int. Rev. Cytology,* 2003, vol. 223, 178-288
- **Hu C-L et al.** *Biol Reprod,* 2001, vol. 64, 518-526
- **Cristofalo VJ ; Pignolo RJ.** *Physiol. Reviews,* 1993, vol. 73, 617-638
- **Dimri et al.** *Proc. Nat. Acad. Sci.,* 1995, vol. 92, 9363-9367